# EUROPEAN PATENT APPLICATION

(11) **EP 2 392 656 A1**
(43) Date of publication of application: **07.12.2011**
(21) Application number: 10735812.9
(22) Date of filing: 26.01.2010
(51) Int. Cl.: C12N 15/09, C12N 1/19, C12P 21/00

(54) **METHOD FOR TRANSFORMING SCHIZOSACCHAROMYCES POMBE, TRANSFORMANT OF SCHIZOSACCHAROMYCES POMBE, AND METHOD FOR PRODUCING HETEROLOGOUS PROTEIN**

(30) Priority: 27.01.2009 JP 2009015472
(71) Applicant: Asahi Glass Company, Limited, Chiyoda-ku Tokyo 100-8405 (JP)
(72) Inventor: IDIRIS, Alimjan, Tokyo 100-8405 (JP); TOHDA, Hideki, Tokyo 100-8405 (JP); HAMA, Yuko, deceased (JP)
(74) Representative: Müller-Boré & Partner Patentanwälte
(86) International application number: PCT/JP2010/050984
(87) International publication number: WO 2010/087344

(57) **Abstract**

To provide a method for transforming *S.pombe* for creating a transformant with a high stability of maintenance after passage and enables the steady production of a heterologous protein of interest, a transformant produced by the method and a method for producing a heterologous protein using the resultant transformant.

A method for transforming *Schizosaccharomyces pombe* by using a vector carrying an expression cassette (containing a promoter capable of functioning in *Schizosaccharomyces pombe,* a heterologous protein structural gene and a terminator), and having recombination region(s) at which homologous recombination with each chromosome of *Schizosaccharomyces pombe* is to be achieved, which comprises integrating the expression cassette into the Tf2 transposon gene position(s) in the chromosome(s) of *Schizosaccharomyces pombe* by homologous recombination, a transformant created by the method and a method for producing a heterologous protein using the resultant transformant.

## Description

### TECHNICAL FIELD

The present invention relates to a method for transforming *Schizosaccharomyces pombe,* a transformant of *Schizosaccharomyces pombe* and a method for producing a heterologous protein.

### BACKGROUND ART

The gene recombination technology is widely applied in various industries for production of heterologous proteins, which are not produced inherently by the host, by using transformants, in which foreign genes (heterologous protein structural genes) encoding the target proteins are introduced. In such heterologous protein production, particularly in the case of producing heterologous proteins derived from eukaryotic microorganisms, use of eukaryotic microorganisms as hosts has been considered to be a good method. Particularly, because yeasts do not contain substances harmful to humans and are easy to handle as unicellular organisms, a lot of expression systems using yeasts as the host have been developed.

Among yeasts, a fission yeast *Schizosaccharomyces pombe* (hereinafter referred to as *S.pombe*) is closer to animal cells than other yeasts inclusive of a budding yeast *Saccharomyces cerevisiae* in various properties such as cell cycle, chromosomal structure and RNA splicing, and hence, can produce proteins with more complex post-translational modifications similar to those in animal cells.

In the production of heterologous proteins using the *S.pombe* as the host, expression vectors (each vector contains a heterologous protein structural gene, a promoter and a terminator) are usually introduced to *S.pombe* as an extrachromosomal gene (a plasmid). However, in such a case, the expression vectors are likely to drop off and disappear from the *S.pombe* transformant cells during cultivation, and therefore it is required to retain the plasmids in each cell by using auxotrophic complementation or drug resistance. Therefore, for more stable production of heterologous proteins, use of *S.pombe* transformants, in which the expression vectors were integrated into their chromosomes by homologous recombination, was proposed (Patent Document 1).

### PRIOR ART DOCUMENT

### PATENT DOCUMENT

Patent Document 1: JP-A-2000-262284

### DISCLOSURE OF INVENTION

### TECHNICAL PROBLEM

In the transformant described in Patent Document 1, the expression vector integrated into a chromosome is retained after 50 generations. However, in industrial production of heterologous proteins, stable production of the heterologous proteins by longer cultivation process has been required, and therefore transformants having a high passage stability have been desired . The object of the present invention is to provide a method for transforming *S.pombe* to produce a transformant which has a high passage stability and enables the steady production of a heterologous protein of interest, a transformant produced by the method and a method for producing a heterologous protein using the transformant.

### SOLUTION TO PROBLEM

The transformation method for *S.pombe,* the transformant and the method for producing a heterologous protein using the transformant of the present invention are shown below.
[1] A method for transforming *Schizosaccharomyces pombe* by using a vector carrying an expression cassette containing a promoter capable of functioning in *Schizosaccharomyces pombe,* a heterologous protein structural gene and a terminator and having recombination region(s) at which homologous recombination with each chromosome of *Schizosaccharomyces pombe* is to be achieved, which comprises integrating the expression cassette into the chromosome(s) of *Schizosaccharomyces pombe* by homologous recombination, and is **characterized in that** each recombination region has a nucleotide sequence which can induce homologous recombination with target sites which have substantially the same nucleotide sequence as the recombination region and exist separately in different chromosomes of *Schizosaccharomyces pombe* and/or at a plurality of positions of the same chromosome separated by at least one essential gene.
[2] The method for transforming *Schizosaccharomyces pombe* according to the above [1], wherein the target sites exist in at least five positions in the chromosome(s) of *Schizosaccharomyces pombe.*
[3] The method for transforming *Schizosaccharomyces pombe* according to the above [1] or[2], wherein the target sites have a nucleotide sequence which exists in the Tf2 transposon gene(s) .
[4] A method for transforming *Schizosaccharomyces pombe* by using a vector carrying an expression cassette containing a promoter capable of functioning in *Schizosaccharomyces pombe,* a heterologous protein structural gene and a terminator, and having recombination region(s) at which homologous recombination with chromosome(s) of *Schizosaccharomyces pombe* is to be achieved, which comprises integrating the expression cassette into the chromosome(s) of *Schizosaccharomyces ρombe* by homologous recombination, and is **characterized in that** the target sites in the chromosome(s) of *Schizosaccharomyces pombe* have a nucleotide sequence which exists in the Tf2 transposon gene(s).
[5] The method for transforming *Schizosaccharomyces pombe* according to any one of the above [1] to [4], wherein a transformant having the expression cassette integrated into at least two positions of the target sites is selected.
[6] The method for transforming *Schizosaccharomyces pombe* according to any one of the above [1] to [5], wherein the vector has at least two copies of the expression cassette.
[7] The method for transforming *Schizosaccharomyces pombe* according to any one of the above [1] to [6], wherein a transformant in which a total of from 3 to 40 copies of the expression cassette have been integrated into the chromosome(s) after the homologous recombination is selected.
[8] The method for transforming *Schizosaccharomyces pombe* according to any one of the above [1] to [7], wherein the expression cassette has a secretion signal gene capable of functioning in *Schizosaccharomyces pombe* at the 5' end of the heterologous protein structural gene in the expression cassette.
[9] A transformant obtained by the transformation method as defined in any one of the above [1] to [8].
[10] A method for producing a heterologous protein which comprises incubating the transformant as defined in the above [9] and obtaining the heterologous protein produced by the transformant.
[11] A method for producing a heterologous protein which comprises incubating a transformant obtained by the transforming method as defined in the above [8] in a culture medium and obtaining the heterologous protein produced by the transformant from the culture medium.

### ADVANTAGEOUS EFFECTS OF INVENTION

According to the method for transforming *S.pombe* of the present invention, it is possible to obtain a transformant which can be passaged more stably and can produce a heterologous protein of interest stably.

Further, the transformant of the present invention can be passaged more stably and can produce a heterologous protein of interest more stably.

Further, the method for producing a heterologous protein of the present invention uses a transformant which can be passaged more stably, and hence, can produce a heterologous protein of interest more stably.

### BRIEF DESCRIPTION OF DRAWINGS

[Fig. 1] A scheme figure showing distribution of Tf2 gene on the chromosomes.
[Fig. 2] A structure map of the Tf2 integrated vector.
[Fig. 3] An electropherogram showing a copy number of the integrated gene and a position of integration.
[Fig. 4] A graph showing a comparison of the stability after 50 passages.

### DESCRIPTION OF EMBODIMENTS

### [Host]

The transformant of the present invention is obtained by using *S.pombe* as the host and integrating an expression cassette containing a promoter capable of functioning in *S.pombe,* a heterologous structural gene (a gene coding a heterologous protein) and a terminator into the chromosomes of the host. *S.pombe* to be used in the present invention may be a wild-type or a mutant-type in which a specific gene is deleted or inactivated depending on application. For deletion or inactivation of a specific gene, conventional methods can be used. Specifically, the Latour system (Nucleic Acids Res. (2006) 34: e11, and WO2007/063919) can be used to delete the gene. Further, the gene can be inactivated by mutating the gene at a certain position by mutant screening using mutagens (Koubo Bunshi Idengaku Jikken-Hou, 1996, Japan Scientific Societies Press), random mutations using PCR (polymerase chain reaction) (PCR Methods Appl., 1992, vol. 2, p.28-33) and the like. As the *S.pombe* host in which a specific gene is deleted or inactivated, ones disclosed in WO2002/101038, WO2007/015470, etc. may, for example, be used.

Further, it is preferred to use a *S.pombe* host having a marker for selecting a transformant. For example, it is preferred to use a host which essentially requires a specific neutrient factor for growth due to deletion of a gene. By using a vector carrying the deleted gene (auxotrophic complementation marker), a transformant lacking the auxotrophy of the host will be obtained. It is possible to select the transformant by using the difference in auxotrophy between the host and the transformant.

For example, a *S.pombe* host which has been made auxotrophic for uracil by deletion or inactivation of orotidine-phosphate decarboxylase (ura4 gene) is transformed with a vector containing ura4 gene (auxotrophic complementation marker), and transformants carrying the vector are obtained by selecting ones lacking uracil auxotrophy. The gene to be deleted to make an auxotrophic host is not limited to ura4 gene when it is used for selection of a transformant, and may, for example, be isopropyl malate dehydrogenase gene (leu1 gene).

### [Vector]

The vector of the present invention has an expression cassette containing a promoter capable of functioning in *S.pombe,* a heterologous structural gene and a terminator, and has a recombination region at which homologous recombination with chromosome of *S.pombe* is to be achieved. Such an expression cassette is a combination of DNA which is required for expression of a heterologous protein encoded by the heterologous protein structural gene, and contains a heterologous protein structural gene, a promoter capable of functioning in *S.pombe* and a terminator.

The expression cassette may additionally have at least one of a 5'-untranslated region and a 3'-untranslated region. The expression cassette preferably has all of the heterologous protein structural gene, the promoter, the terminator, the 5'-untranslated region and the 3'-untranslated region. Further, genes such as an auxotrophic complementation marker and a secretion signal gene which is capable of functioning in *S.pombe* and is introduced at the 5' end of the heterologous protein structural gene, may be contained.

The heterologous protein structural gene is a gene encoding a heterologous protein. However, the heterologous protein of the present invention means a protein which *S.pombe* does not inherently produce (which is not encoded by any gene in wild-type *S.pombe*). The heterologous protein is not particularly limited, but is preferably a protein produced by human or any other animals as a pharmaceutical raw material, a protein produced by microorganisms as an enzyme for industrial use or a protein produced by plants as a raw material for food, in view of its excellent industrial values.

The promoter and the terminator may be ones capable of functioning in a *S.pombe* host to direct expression of a heterologous protein. As the promoter capable of functioning in *S.pombe,* a promoter intrinsic to *S.pombe* (preferably one having a high transcriptional activity) or a promoter extrinsic to *S.pombe* (such as a promoter derived from a virus) may be used. Further, two or more types of promoters may be contained in the vector.

As the promoter intrinsic to *S.pombe,* a promoter of alcohol dehydrogenase gene, a promoter of nmt1 gene which relates to thiamine metabolism, a promoter of fructose 1,6-bisphosphatase gene which relates to glucose metabolism, a promoter of an invertase gene which relates to catabolite repression (WO99/23223) or a promoter of a heat shock protein gene (WO2007/26617) may, for example, be mentioned.

As the promoter extrinsic to *S.pombe,* the promoter disclosed in JP-A-5-15380, JP-A-7-163373 or JP-A-1 0-234375 may, for example, be mentioned, and CMV promoter or SV40 promoter is preferred.

As the terminator capable of functioning in *S.pombe,* a terminator intrinsic to *S.pombe* or a terminator extrinsic to *S.pombe* may be used. Further, two or more types of terminators may be contained in the vector.

As the terminator, the terminator derived from the human disclosed in JP-A-5-15380, JP-A-7-163373 or JP-A-10-234375 may, for example, be mentioned, and preferably human lipocortin-I terminator.

The vector of the present invention preferably contains a selection marker. For example, a vector carrying an auxotrophic complementation marker proper for the auxotrophy of the host is preferably used.

The secretion signal gene capable of functioning in *S.pombe* is a gene having a function of secreting the expressed heterologous protein out of the host cell. A heterologous protein to which the secretion signal is bound at the N-terminal is expressed from a heterologous protein structural gene to which the secretion signal gene is bound, and then the secretion signal is removed from the protein in the endoplasmic reticulum and the Golgi apparatus, etc. Thereafter, the heterologous protein detached from the secretion signal is secreted out of the cell. The secretion signal gene (and the secretion signal) should be capable of functioning in *S.pombe.* As the secretion signal gene capable of functioning in *S.pombe,* the secretion signal genes described in WO1996/23890 may be used.

The vector of the present invention is a vector having a circular DNA structure or a linear DNA structure, and is preferably introduced to *S.pombe* cells in the form of a linear DNA structure. That is, in a case where a vector having a circular DNA structure like a usual plasmid DNA is used, it is preferred that the vector is cut open to a linear form by a restriction enzyme before introduction to the *S.pombe* cells. In this case, the vector having a circular DNA structure is cut open at a position within the recombination region. The resulting vector has parts of the recombination regions exist at both ends and is integrated entirely into the target site of a chromosome by homologous recombination.

The vector may be constructed by other methods without cutting a vector having a circular DNA structure, for example, by enzymatic amplification by PCR or a chemical synthesis, so long as a linear DNA structure having parts of the recombination region at both ends can be obtained.

To construct the vector of the present invention, a plasmid derived from E. *coli* such as pBR322, pBR325, pUC118, pUC119, pUC18, pUC19 or the like may suitably be used. A vector constructed by using a plasmid derived from E. *coli* usually has the replication origin region called "ori" which is necessary for replication in *E. coli.* Further, even in a case where a plasmid derived from *E. coli* like those mentioned above is not used, when *E. coli* is used for construction and amplification of the vector of the present invention, the "ori" is utilized to obtain a vector having "ori".

In this case, it is preferred that the replication origin region called "ori" required for replication in E. *coli* is removed from the vector for homologous recombination. Accordingly, it is possible to improve the integration efficiency (JP-A-2000-262284) at the time of integrating the above-described vector into a chromosome.

The method for constructing the vector in which the replication origin region is removed is not particularly limited, but is preferably the method disclosed in JP-A-2000-262284. That is, it is preferable to preliminarily construct a precursor vector carrying the replication origin region at a position to be cut within the recombination region so that the replication origin region will be cut-off from the vector at the time of preparing a linear DNA structure. Thus, a vector in which the replication origin region is removed is obtained easily.

Further, it may be a method wherein a precursor vector containing an expression cassette and a recombination region is constructed by using the vectors and their construction method disclosed in JP-A-5-15380, JP-A-7-163373, WO96/23890, JP-A-10-234375, and then the replication origin region is removed from the precursor vector by using a usual genetic engineering method to obtain a vector to be used for homologous recombination.

The number of copies of the expression cassette in the vector may be only one, or two or more. In a case where the number of copies of the expression cassette in the vector is one, two or more copies of the expression cassette may be integrated in the same position of the *S.pombe* chromosome. Further, in a case where the number of copies of the expression cassette in the vector is two or more, a plurality of the expression cassettes may be integrated sequentially in the same position of the *S.pombe* chromosome.

The number of copies of the expression cassette in the vector of the present invention is preferably from 1 to 8, particularly preferably from 2 to 4.

When the number of copies of the expression cassette in the vector is at least 2, it becomes easier to increase the number of copies of the expression cassette integrated into the *S.pombe* chromosome and expression of a heterologous protein. However, in a case where the number of the after-mentioned target site is large, many copies of the expression cassette can be integrated in the present invention even if the vector has one copy of the expression cassette. Further, when the number of copies of the expression cassette in the vector is at most 8, reduction in the efficiency of vector integration via homologous recombination, which happens in a case where the vector size is too large, is likely to be suppressed. When the number of copies of the expression cassette is at most 4, the vector integration efficiency can be improved further.

The recombination region of the vector is a region having a nucleotide sequence which can induce homologous recombination with a target site in the *S.pombe* chromosome at which homologous recombination is to be achieved. Further, the target site is a site to become a target for integration of an expression cassette in the *S.pombe* chromosome. The target site can be designed freely by letting the recombination region of the vector have a nucleotide sequence which induce homologous recombination with the target site.

To induce homologous recombination, the recombination region is required to have a nucleotide sequence with a homology of at least 70% with the target site. Further, the nucleotide sequence homology between the recombination region and the target site is preferably at least 90%, more preferably at least 95%, in view of increasing the efficiency of homologous recombination. By using a vector having such a recombination region, the expression cassette is integrated into the target site by homologous recombination.

The length of the recombination region (the number of nucleotides) is preferably from 20 to 2,000 bp. When the length of the recombination region is at least 20 bp, homologous recombination is likely to be induced. Further, when the length of the recombination region is at most 2,000 bp, reduction in the homologous recombination efficiency due to too a large vector size is likely to be prevented. The length of the recombination region is preferably at least 100 bp, more preferably at least 200 bp. Further, the length of the recombination region is preferably at most 800 bp, more preferably at most 400 bp.

### [Target site of host]

The target site for integration of the expression cassette is a plurality of target sites in all three chromosomes of *S.pombe,* which satisfies either one of conditions (1) two or more target sites exist in different chromosomes or (2) two or more target sites exists at a plurality of positions in the same chromosome separated by at least one essential gene, or both of (1) and (2). These two or more target sites have substantially the same nucleotide sequence. Here, "substantially the same nucleotide sequence" means that there is at least 90% nucleotide sequence homology between target sites. The nucleotide sequence homology between target sites is preferably at least 95%, more preferably at least 99%.

The above-described essential gene means a gene whose lost or inactivation leads to inviability, i.e. a gene essential for growth of the transformant of the present invention. Therefore, when the transformant loses the introduced expression cassettes which are separated by the essential gene, the essential gene is also lost, whereby the transformant cannot grow. Accordingly, during incubation, the transformant which lost the expression cassettes may not grow along with the transformant retaining the expression cassettes, whereby the heterologous protein production efficiency is unlikely to be reduced (because the growth rate of the transformant which lost expression cassettes has a higher growth rate in general).

As described above, since the target sites are dispersed in the chromosomes of *S.pombe,* the expression cassettes are integrated into the chromosome in a dispersed manner, and then the integrated expression cassettes are unlikely to be lost and the passage is quite high. Accordingly, it is possible to produce a heterologous protein stably with high productivity.

Further, by designing the target site so as to have substantially the same nucleotide sequence, even in a case where a plurality of target sites exist in different positions, it becomes possible to easily integrate the vector into the respective target sites by using a single type of vector.

In the transforming method of the present invention, the number of target site positions where the vector is to be integrated is preferably at least 5. When the number of target site positions is at least 5, it is easier to increase the number of copies of the expression cassette integrated into the chromosome, whereby the productivity of a heterologous protein increases further.

Further, the number of target site positions is preferably from 10 to 60. When the number of target site positions is at least 10, it is easier to further increase the number of copies of the expression cassette integrated into the chromosome, whereby the productivity of a heterologous protein increases further. When the number of target site positions is at most 60, reduction in expression of a heterologous protein due to integration of too many copies of the expression cassettes into the chromosome is likely to be suppressed.

The target sites preferably have a nucleotide sequence which exists in a transposon gene Tf2 (a transposon gene which has a length (the number of nucleotide) of about 4,900 bp and exists in the three chromosomes at 13 positions in total, with a nucleotide sequence homology of 99.7%. Refer to the following document), since it becomes possible to integrate the expression cassettes into the target sites dispersed at plural positions in the *S.ρombe* chromosomes at a time by using a single type of vector.

### (Document)

Nathan J. Bowen et al, "Retrotransposons and Their Recognition of pol 11 Promoters: A Comprehensive Survey of the Transposable Elements From the Complete Genome Sequence of Schizosaccharomyces pombe", Genome Res. 2003 13: 1984-1997

However, the target sites are not limited to the above-described ones. Other than the transposon gene Tf2, the target sites may, for example, be sites (such as genes) found at plural positions in the chromosomes, and having a length larger than the length of the recombination region and a substantially identical nucleotide sequence. Further, for example, after formation of the target site by newly introducing a plurality of genes (target sites) having a substantially identical nucleotide sequence and a length larger than the length of the recombination region into the chromosomes, the vector may be introduced into a plurality of target sites.

### [Transformant and transformation method]

The method for transforming *S.ρombe* of the present invention comprises integrating the expression cassette into the above-described target sites in the chromosomes of the *S.ρombe* host by homologous recombination, by using the above-described vector. The vector may be integrated into the chromosomes by homologous recombination, by using publicly known methods or well known methods (for example, JP-A-2000-262284). Further, the transformant of the present invention is a transformant obtained by the transforming method.

The above-described vector may be integrated into one or at least two of the plural target sites which exist in the *S.pombe* chromosomes at plural positions. In the present invention, it is preferred to select a transformant having the vector integrated into two or more target sites.

Further, the vector may be integrated into all of the target sites or only some of the plural target sites in the chromosomes. Because if too many expression cassettes are integrated into the chromosomes, expression of the heterologous protein may decrease, it is preferred that the vector is integrated into at most 20 target sites. Therefore, in a case where the number of the target sites which exist in the chromosomes is at most 20, the vector may be integrated into all of the target sites, and in a case where the number of the target sites which exist in the chromosomes is larger than 20, it is preferred that the vector is integrated into at most 20 target sites. It is preferred that the vector is integrated into from 2 to 15 target sites (as many target sites if the number of target sites is less than 15), more preferably from 3 to 10 target sites (as many target sites if the number of the target site is less than 10). In a case where the target site is the transposon gene Tf2 (the number of the target sites is 13), it is preferred that Tf2 is integrated into from 2 to 13 target sites, particularly preferably from 2 to 8 target sites.

As described above, the vector may contain two or more copies of the expression cassettes. Accordingly, the total number of copies of the expression cassette integrated into the chromosomes is the number of copies of the expression cassette in the vector multiplied by the number of the target sites where the vector is integrated. In the transformant obtained by the present invention, the total number of copies of the expression cassette in the chromosomes is preferably from 2 to 40, more preferably from 2 to 20. Because if too many expression cassettes are integrated into the chromosomes, expression of the heterologous protein may decrease, the total number of copies of the expression cassette integrated into the chromosomes is preferably at most 40, more preferably at most 20.

In the transforming method of the present invention, usually after homologous recombination, the resulting transformants are subjected to selection. The selection may, for example, be carried out as follows. Several transformants are selected as viable colonies in a medium via the above-mentioned auxotrophic marker screening method, the transformants are grown separately in a liquid medium, and transformants highly expressing a heterologous protein are selected by measuring expression of a heterologous protein in each culture medium. The number of vectors and copies of the expression cassette integrated into the chromosomes can be identified by analyzing the genomes of the selected transformants by pulse-field gel electrophoresis.

The number of vectors integrated into the chromosomes can be adjusted to some extent by adjusting integration conditions. Further, the integration efficiency and the number of integration vector may be changed also depending on the length (nucleotides) and the structure of vectors.

The object of the present invention is to obtain a transformant having a high expression efficiency of a heterologous protein, not to simply obtain a transformant having many copies of an expression cassette in total. Generally, it can be anticipated that the expression efficiency increases with the number of copies of an expression cassette. However, if the number of copies of an expression cassette is too large, it is possible to think that burdens for survival and growth to cells increases, whereby the expression efficiency of a heterologous protein will be lowered. Further, if the size of vector is too large, the efficiency of chromosomal integration decreases and therefore number of integrated vectors is unlikely to become large, whereby a transformant itself cannot be obtained. The size of vector may be affected not only by the number of copies of an expression cassette but also the length (the number of nucleotide) of a heterologous protein structural gene. Therefore, as compared with a case of a short heterologous protein structural gene, the number of copies of an expression cassette integrated into the chromosomes is restricted in a case of a long heterologous protein structural gene. Further, the heterologous protein structural gene may be changed depending not only on its length but also on the types of the gene and the configuration of its nucleotide sequence.

The transformant of the present invention is a transformant obtained by the above-described transforming method of the present invention. The transformant contains two or more copies of an expression cassette, and has an expression efficiency higher than that of a transformant having one copy of the same expression cassette. The optimal total number of copies of the expression cassette required for obtaining a transformant having a high efficiency of a heterologous protein expression may depend on the length or types of a heterologous protein structural gene, as described above. However, so long as a certain amount of a transformant is produced by homologous recombination, a transformant may be obtained by selection, and at the same time, by measuring the efficiency of heterologous protein expression of the obtained transformant to select a strain having a high efficiency of heterologous protein expression from the obtained transformant. The expression efficiency can be associated with the number of copies of an expression cassette after the above selection and measurement of the number of copies of the expression cassette in the transformant. Provided that one type of a heterologous protein structural gene is an object, usually, the expression efficiency increases as the number of copies of the expression cassette becomes large to some extent. However, in between different types of heterologous protein structural genes, there may be a case in which the efficiency of expression is not associated with the number of copies of the expression cassette.

In the inventors' previous patent (JP-A-2000-262284), a transformant having two or more copies of an expression cassette in chromosomes is provided. However, as compared with a case where a plurality of expression cassettes exits in only one chromosomal position, the transformant of the present invention has a quite high passage stability since the expression cassette is dispersed in chromosomes. For example, a burden to growth or expression may be dispersed comparing to a case where expression cassettes are clustered in one position, and even if lost of expression cassettes occur in one position, expression cassettes in other positions may be remained. Further, even if a transformant having expression cassettes at plural positions by using different vectors (vectors different in nucleotide sequence in their recombination regions) to integrate an expression cassette into two or more types of target sites, it is possible to introduce the expression cassette into plural target sites at the same time by using a single type of vector in the present invention, whereby production of a transformant becomes quite easy.

### [Method for producing heterologous protein]

The method for producing a heterologous protein of the present invention comprises incubating the transformant of the present invention and recovering a heterologous protein produced by the transformant.

As the culture medium, a known culture medium for yeasts may be used as long as it contains carbon sources, nitrogen sources, inorganic salts and the like which *S.ρombe* can use, and *S.pombe* can grow in it efficiently. The culture medium may be natural or synthetic.

As the carbon sources, saccharides such as glucose, fructose and sucrose may, for example, be mentioned.

As the nitrogen sources, inorganic acids or inorganic ammonium salts such as ammonia, ammonium chloride, and ammonium acetate, peptone and casamino acid may, for example, be mentioned.

As inorganic salts, magnesium phosphate, magnesium sulfate and sodium chloride may, for example, be mentioned.

Incubation may be carried out by using a known culture method for yeasts such as a shaking incubation, a stirring incubation or the like.

The incubation temperature is preferably from 23 to 37°C. Further, the incubation time may be set appropriately.

Incubation may be carried out batch-wise or continuously.

Recovery of a heterologous protein may be carried out by using a known protein recovery method. For example, the cells are separated from the culture medium after incubation, and then the separated cells are destroyed to obtain a component containing a heterologous protein of interest, followed by recovery of the heterologous protein of interest from the component by using a known protein isolation method. Further, a transformant obtained by using an expression vector having a heterologous protein structural gene to which a secretion signal gene is attached secretes the heterologous protein into the culture medium. Accordingly, in this case, the heterologous protein of interest can be recovered from the culture medium by using a known protein isolation method.

By using a transformant secreting a heterologous protein to a culture medium and continuous incubation of the transformant, the heterologous protein can be recovered efficiently. The continuous incubation is carried out, for example, by recovering the heterologous protein along with a culture supernatant from the culture medium after a certain period of incubation, and then re-incubating the culture after supplementing the culture supernatant with a culture medium.

### EXAMPLES

Now, the present invention will be described in further detail with reference to specific Examples. However, the present invention is by no means restricted to the following Examples.

### [EXAMPLE 1] Identification of gene loci into which an expression cassette can be integrated by a single transformation

The fission yeast chromosomes are known to have a plurality of highly conserved redundant sequences which may be appropriate for integrating expression cassettes into a plurality of positions at a time, by determination of the nucleotide sequence of the chromosomes (Wood et al., Nature, Vol.415, pp. 871-880, 2002). Then, by using GenDB database of the Sanger Institute (http://www.genedb.org/genedb/), comprehensive identification of gene loci into which an expression cassette can be integrated by a single transformation, was carried out.

At first, a redundancy of a AT-rich sequence was identified in a centromere and a replication origin region. However, these sequences were found to have a poor nucleotide sequence homology, thereby found to be inappropriate for introduction of an exogenous gene.

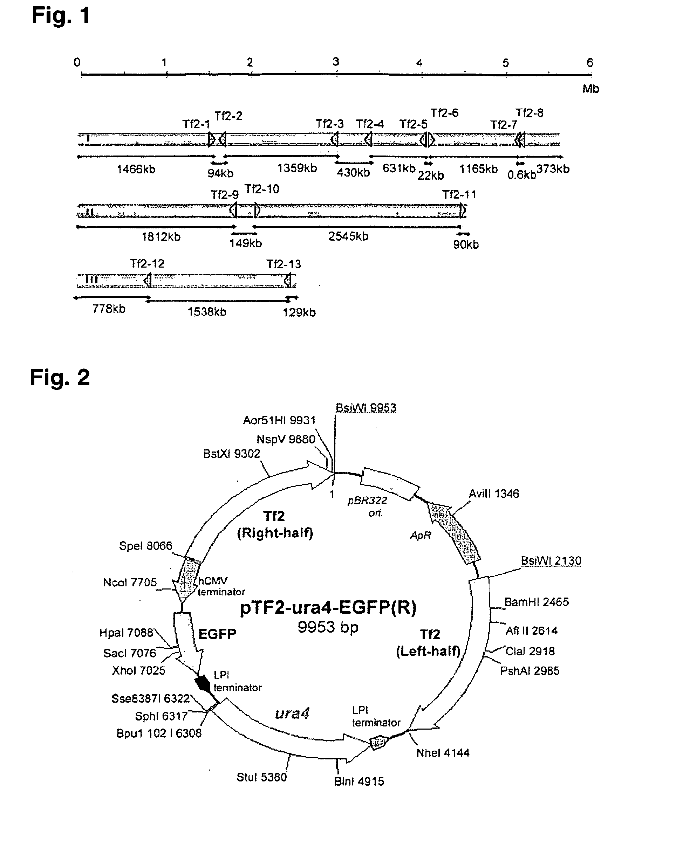

Next, a redundancy of nucleotide sequences derived from tansposons such as LTR and Tf2 were also identified. Although LTRs are known to exist in 174 copies (Bowen et al., Genome Research, Vol. 13, pp. 1984-1993, 2003), their sequence homologies were not high. However, 13 copies of Tf2 elements were found to be dispersed over the entire chromosomes with a sequence homology of at least 99%. Their structures are shown in Figure 1. As shown in Figure 1, except that Tf2-7 and Tf2-8 which were adjacent to each other, Tf2 elements were dispersed over the entire chromosomes, and genes essential for growth were always found in-between those elements. Accordingly, we concluded that it was possible to use these elements as loci for gene integration.

Further, the telomeric chromosomal regions had been estimated to have a redundancy of four homologous genes (Mandell et al., Genome Biology, Vol. 6, page R1, 2004). However, the subtelomeric chromosomal regions were known to suppress gene expression, whereby they were inappropriate for expression of exogenous genes. Although telomere itself is comprised of a redundant nucleotide sequence, its length is supposed to change depending on cell cycle, whereby it was inappropriate for production of a heterologous protein.

### [EXAMPLE 2] Construction of a Tf2 integration type vector

pTf2-ura4-EGFP(R) vector (Figure 2) which can integrate a Green Fluorescent Protein (GFP) expression cassette into Tf2 gene loci was constructed by the following method.

At first, the whole genomic DNA was collected from *S. pombe* cells by using a whole genomic DNA extraction kit (DNeasy manufactured by Qiagen), and then 1 µg was collected as a template for PCR amplification of the DNA fragment (about 3,950 bp) of *S. pombe* Tf2-2 (GeneDB systematic name: SPAC167.08) by using a pair of primers, 5'-AAGGCCTCGTACGTGAAAGCAAGAGCAAAACGA-3' and 5'-AAGGCCTCGTACGTGCTTTGTCCGCTTGTAGC-3', each having a restriction enzyme BsiWI recognition sequence (CCTACG) at the 5' end side. After digestion of both ends with restriction enzyme BsiWl , the amplified DNA fragment was separated and purified by agarose gel electrophoresis to prepare an insert fragment.

Next, the chromosomal integration vector pXL4 (Idiris et al., Yeast, Vol. 23, pp. 83-99, 2006) was digested with the same restriction enzyme BsiWl to prepare a region (about 2,130 bp) containing an ampicillin resistant gene (ApR) and a replication origin of E. *coli* (pBR 322 ori). The DNA fragment was further treated with a dephosphorylase (CIAP, manufactured by Takara Bio Co., Ltd.) for dephosphorylation, and then separated and purified by agarose gel electrophoresis to prepare a vector fragment.

Ligation of the insert fragment and the vector fragment was carried out by using a ligation kit (DNA Ligation Kit ver. 2, manufactured by Takara Bio Co., Ltd.), followed by transformation of E. *coli* DH5 (Toyobo Co., Ltd.) to construct a recombination plasmid pTf2-2 (6,071 bp).

By using 0.1µg of the above-constructed vector pTf2-2 as a template and a primer pair comprised of a primer 5'-GGGGTACCAAGCTTCTAGAGTCGACTCCGGTGCTACGACACTTT-3' (which has recognition sequences for Kpnl, Hindlll, Xbal and SalI at the 5' end) and a primer 5'-GGGGTACCAGGCCTCTCGAGGCTAGCCATTTCCAGCGTACATCCT-3' (which has recognition sequences for Kpnl, StuI, Xhol and Nhel at the 5' end) for PCR amplification, a fragment having the whole length of 6,060 bp was obtained. After Kpnl digestion of both ends, the fragment was separated and purified by agarose gel electrophoresis, followed by self ligation by using a ligation kit to prepare pTf2 (MCS) vector having a length of 6,058 bp and a multiple cloning site (MCS) within the nucleotide sequence of Tf2-2 retrotransposon.

The above-constructed pTf2 (MCS) vector was subjected to double digestion with restriction enzymes Kpnl and Nhel, and then separated and purified by agarose gel electrophoresis to prepare a 6,040-bp fragment. Further, a fragment having recognition sequences for restriction enzymes Kpnl and Nhel at both ends of *S. pombe* uracil-auxotrophy marker ura4 (orotidine-5'-phosphate decarboxylase gene, GeneDB systematic name: SPCC330.05c) which were introduced by using PCR, was prepared, and subjected to double digestion with restriction enzymes Kpnl and Nhel, and then separated and purified by agarose gel electrophoresis to obtain a 2,206-bp fragment. The two fragments obtained were ligated by using a ligation kit to prepare pTf2 (MCS)-ura4 vector having a length of 8,246 bp and a multiple cloning site (MCS) within the nucleotide sequence of Tf2-2 retrotransposon.

The pTf2 (MCS)-ura 4 vector constructed above was digested with restriction enzyme Sall. After blunting of both ends by using a blunting kit (DNA Blunting kit, manufactured by Takara Bio Co., Ltd.), the resulting 8,246-bp fragment was separated and purified by agarose gel electrophoresis, followed by dephosphorylation to prepare a vector fragment. Further, pTL2EGFP1 was subjected to double digestion with restriction enzymes Spel and Bst11071 to cut off a GFP expression cassette having a length of 1,720 bp. After blunting of both ends by using a blunting kit, the GFP expression cassette was separated and purified by agarose gel electrophoresis to prepare an insert fragment. The vector and the insert fragment were ligated by using a ligation kit to prepare Tf2 integration vector pTf2-ura4-EGFP(R) (Figure 2).

### [EXAMPLE 3] Preparation of a transformant

3 µg of the Tf2 integration vector pTf2-ura4-EGFP(R) prepared in EXAMPLE 2 was digested with restriction enzyme BsiWl. By using the entire amount of the digest, fission yeast ARC010 strain (Genotype: *h⁻ leul-32 ura4-D18*) was transformed in accordance with a method of Okazaki et al. (Nucleic Acids Research, Vol. 18, pp. 6485-6489, 1990). The transformant was plated onto the surface of a minimal medium supplemented with leucine (MMA+leu) and incubated at 32°C for 4 days. Among about 500 uracil-prototrophic colonies grown on the medium, 96 colonies showing fluorescence from green fluorescent protein (GFP) were selected by visual observation using a fluorescence observation apparatus (GFP Viever TR-1 00, Ikeda Scientific Co., Ltd.).

Each colony was scraped off from the medium, then inoculated in 1 ml of leucine-less SDC-Leu medium (SD Medium-LEU, manufactured by Qbiogene Inc.) and incubated for about 24 hours with shaking in a 24-well culture plate. 0.1 ml of the each cell culture was collected, then inoculated in 1 ml of YES liquid culture medium (5 g/l of yeast extract (Becton, Dickinson and Company), 30 g/l of glucose and 250 mg/l of SP supplements (Qbiogene Inc.)) and incubated for about 24 hours with shaking in a 24-well culture plate. Then, by using a fluorescence intensity measuring device (SpectraMax Gemini XS, manufactured by Molecular Devices Ltd.), a fluorescence intensity per 1 ml of the liquid cell culture was measured, and a clone having the highest GFP fluorescence intensity was selected.

### [EXAMPLE 4] Analysis of integration positions and copy numbers over the chromosomes

The whole DNA fraction was extracted from the transformant prepared in EXAMPLE 3. The universal primer for the vector side 5'-GGTGATGACGGTGAAAACCT-3' and each of 13 primer pairs specific for Tf2 gene listed in Table 1 were mixed, and PCR amplification was carried out by using the extracted DNA as a template and KOD-Plus (Toyobo Co., Ltd.) to analyze positions of the integrated expression cassette and their copy numbers.

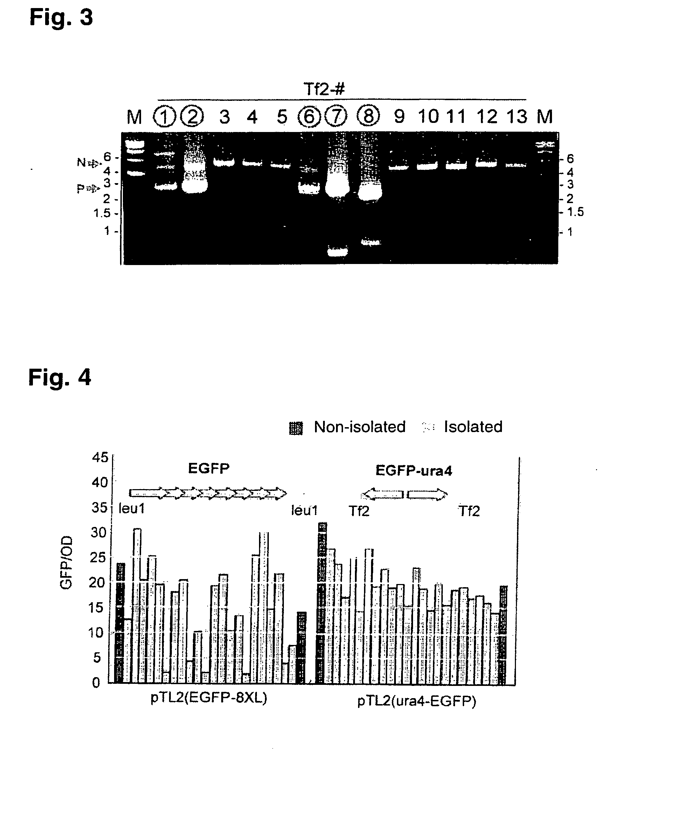

As a result, as shown in Figure 3, it was found five copies of the expression cassette were integrated into five positions of Tf2-1, Tf2-2, Tf2-6, Tf2-7 and Tf2-8.

**TABLE 1**

| Target genes | Primer pairs | | PCR band (negative) | PCR band (positive) |
|---|---|---|---|---|
| | Forward primers | Reverse primers | | |
| Tf2-1 | 5'-GTGGCGGAGGATAAACTGAA-3' | 5'-AAGCATAGCCGAACAAATCC-3' | 5.4 kbp | 2.8~2.9 kbp |
| Tf2-2 | 5'-GGGCAATTTATGGTGGTGAC-3' | 5'-ATTGGTTGTTCAATGCCAAA-3' | 5.2 kbp | 2.6~2.7 kbp |
| Tf2-3 | 5'-TTGAAACGCGTCTGTCTTTG-3' | 5'-TCGGCTCGTTAAGCATCTTT-3' | 5.3 kbp | 2.7~2.8 kbp |
| Tf2-4 | 5'-TCGCTTTTTCTGACAGCTCA-3' | 5'-AAAGTGCTGGAACACCCATT-3' | 5.3 kbp | 2.7~2.8 kbp |
| Tf2-5 | 5'-CGTTGGCGGACGTAGTAAAG-3' | 5'-AGCATTGCATACCCTAAAGCA-3' | 5.0 kbp | 2.5~2.7 kbp |
| Tf2-6 | 5'-TTCCAGAAATCCAGAAAGAAGC-3' | 5'-ATTTGCCGTCACCAATTAGC-3' | 5. kbp | 2.5~2.8 kbp |
| Tf2-7 | 5'-CTACAAGCGGACAAAGCAAT-3' | 5'-TGCAAAGGGGTAAAGATCCA-3' | 5.3 kbp | 2.7~2.8 kbp |
| Tf2-8 | 5'-TGCGTCATGTGATGGAAACTA-3' | 5'-CCTTTGAACCCAGAAGGAGA-3' | 5.4 kbp | 2.6~3.0 kbp |
| Tf2-9 | 5'-GTGCATTCCTGTGCATCATT-3' | 5'-ACAAACAAAACGGGCAAAAG-3' | 5.2 kbp | 2.7~2.8 kbp |
| Tf2-10 | 5'-GCTGCTACGTTCATTTTCGT-3' | 5'-GGCGTTATCAAGCTACGGAAC-3' | 5.2 kbp | 2.6~2.8 kbp |
| Tf2-11 | 5'-TCTTCAATGCCTGCTGTTCA-3' | 5'-CCCTCCCTTGATCACAACAT-3' | 5.1 kbp | 2.6~2.8 kbp |
| Tf2-12 | 5'-CATCCCAACGCATCGTTAAT-3' | 5'-CCACTAAGCCGAACCAAAGA-3' | 5.2 kbp | 2.7~2.8 kbp |
| Tf2-13 | 5'-TTCACCGTCCTATTCCTGAGA-3' | 5'-TCTGATGAGGGTGTTGAACG-3' | 5.1 kbp | 2.6~2.8 kbp |

### [EXAMPLE 5] Comparison in the passage stability of the expression cassettes

The transformant prepared in EXAMPLE 3 was compared with the strain disclosed in JP-A-2000-262284 in the passage stability of an expression cassette.

These two strains were incubated as the objects of evaluation in 5 ml of YES liquid medium (test tube) once, then incubated in 50 ml of YES liquid medium (Erlenmeyer flask) twice, and spread on the YES solid medium for clonal isolation. Twenty two isolated clones were randomly picked up for each strain, inoculated in 1 ml of YES liquid medium, and then incubated for about 24 hours with shaking in a 24-well culture plate. As a control, two clones were picked up for each strain before isolation and incubated at the same time. Thereafter, 0.1 ml of a liquid cell culture was collected for each clone, then inoculated in 1 ml of fresh YES liquid medium and incubated for about 24 hours with shaking in a 24-well culture plate. Finally, by using a fluorescence intensity measuring device (SpectraMax Gemini XS, manufactured by Molecular Devices Ltd.), a fluorescence intensity per 1 ml of a liquid cell culture was measured to obtain a GFP fluorescence intensity distribution.

The results are shown in Figure 4. The fluorescence intensity per OD 660 of the transformant ( pTL2 (ura4-EGFP)) prepared in EXAMPLE 3 was 21.1, and was found to be almost the same as that of the strain ( pTL2 (EGFP-8XL)) disclosed in JP-A-2000-262284, 21.2. However, as shown in Figure 4, whereas the former one had a standard deviation of ± 11 %, the latter one had a standard deviation of 30%, and therefore the expression cassette integrated by the method of the present invention was maintained more stably over passages.

### INDUSTRIAL APPLICABILITY

By using the transformant obtained by the method of the present invention, it is possible to produce a heterologous protein of interest stably with high productivity. Therefore, it can be suitably applied in various industries for production of a heterologous protein.

The entire disclosure of Japanese Patent Application No. 2009-015472 filed on January 27, 2009 including specification, claims, drawings and summary is incorporated herein by reference in its entirety.

## Claims

1. A method for transforming *Schizosaccharomyces pombe* by using a vector carrying an expression cassette containing a promoter capable of functioning in *Schizosaccharomyces pombe,* a heterologous protein structural gene and a terminator and having recombination region(s) at which homologous recombination with each chromosome of *Schizosaccharomyces pombe* is to be achieved, which comprises integrating the expression cassette into the chromosome(s) of *Schizosaccharomyces pombe* by homologous recombination, and is **characterized in that** each recombination region has a nucleotide sequence which can induce homologous recombination with target sites which have substantially the same nucleotide sequence as the recombination region and exist separately in different chromosomes of *Schizosaccharomyces pombe* and/or at a plurality of positions of the same chromosome separated by at least one essential gene.

2. The method for transforming *Schizosaccharomyces pombe* according to Claim 1, wherein the target sites exist in at least five positions in the chromosome(s) of *Schizosaccharomyces pombe.*

3. The method for transforming *Schizosaccharomyces pombe* according to Claim 1 or 2, wherein the target sites have a nucleotide sequence which exists in the Tf2 transposon gene(s) .

4. A method for transforming *Schizosaccharomyces pombe* by using a vector carrying an expression cassette containing a promoter capable of functioning in *Schizosaccharomyces pombe,* a heterologous protein structural gene and a terminator, and having recombination region(s) at which homologous recombination with chromosome(s) of *Schizosaccharomyces pombe* is to be achieved, which comprises integrating the expression cassette into the chromosome(s) of *Schizosaccharomyces pombe* by homologous recombination, and is **characterized in that** the target sites in the chromosome(s) of *Schizosaccharomyces pombe* have a nucleotide sequence which exists in the Tf2 transposon gene(s).

5. The method for transforming *Schizosaccharomyces pombe* according to any one of Claims 1 to 4, wherein a transformant having the expression cassette integrated into at least two positions of the target sites is selected.

6. The method for transforming *Schizosaccharomyces pombe* according to any one of Claims 1 to 5, wherein the vector has at least two copies of the expression cassette.

7. The method for transforming *Schizosaccharomyces pombe* according to any one of Claims 1 to 6, wherein a transformant in which a total of from 3 to 40 copies of the expression cassette have been integrated into the chromosome(s) after the homologous recombination is selected.

8. The method for transforming *Schizosaccharomyces pombe* according to any one of Claims 1 to 7, wherein the expression cassette has a secretion signal gene capable of functioning in *Schizosaccharomyces pombe* at the 5' end of the heterologous protein structural gene in the expression cassette.

9. A transformant obtained by the transformation method as defined in any one of Claims 1 to 8.

10. A method for producing a heterologous protein which comprises incubating the transformant as defined in Claim 9 and obtaining the heterologous protein produced by the transformant.

11. A method for producing a heterologous protein which comprises incubating a transformant obtained by the transforming method as defined in Claim 8 in a culture medium and obtaining the heterologous protein produced by the transformant from the culture medium.
